# EUROPÄISCHE PATENTSCHRIFT

(11) **EP 0 707 247 B1**
(45) Veröffentlichungstag und Bekanntmachung des Hinweises auf die Patenterteilung: **07.02.2007**
(21) Anmeldenummer: 95113811.4
(22) Anmeldetag: 02.09.1995
(51) Int. Cl.: G05B 13/02, G01N 33/18

(54) **Analysegerät, insbesondere für Abwasser**
Analyzer, in particular for waste water
Dispositif d'analyse, en particulier pour les eaux usées

(30) Priorität: 11.10.1994 DE 4436155; 09.06.1995 DE 19521079
(43) Veröffentlichungstag der Anmeldung: 17.04.1996
(60) Teilanmeldung: 05004969.1
(73) Patentinhaber: Endress + Hauser Conducta Gesellschaft für Mess- und Regeltechnik mbH + Co.KG., D-70839 Gerlingen (DE)
(72) Erfinder: Babel, Wolfgang, Dr., D-71263 Weil der Stadt (DE)
(74) Vertreter: Hahn, Christian

(56) Entgegenhaltungen:
- EP-A- 0 417 571
- EP-A- 0 431 910
- EP-A- 0 432 267
- EP-A- 0 521 643
- DE-A- 4 227 727
- MEASUREMENT SCIENCE AND TECHNOLOGY, Bd. 1, Nr. 5, Mai 1990 BRISTOL GB, Seiten 446-451, XP 000116160 J. W. GARDNER ET AL.
- SENSORS AND ACTUATORS B, Bd. b9, Nr. 1, Juli 1992 LAUSANNE CH, Seiten 9-15, XP 000297731 J. W. GARDNER ET AL.

## Beschreibung

Die Erfindung betrifft ein Analysegerät zur Überwachung von Umweltprozessen umfassend mehrere Sensoren, deren Ausgangssignale jeweils einer Schaltungsanordnung zugeführt sind.

Die Überwachung von kommunalen und industriellen Anlagen mit umweltgefährdendem Potential ist ein aktuelles Problem, dessen Bedeutung immer mehr in den Vordergrund rücken wird. Generell wird die Aufgabe bestehen, die unvermeidbare Belastung der Umwelt örtlich und zeitlich im Sinne einer Minimierung zu steuern. Die Überwachung von Umweltprozessen als Basis für gezielte Prozeßeingriffe ist charakterisiert durch die Notwendigkeit einer schnellen synchronen Datenerfassung sowie der Verarbeitung in Echtzeit. Dabei handelt es sich um unsichere, stark gestörte und teilweise widersprüchliche Daten.

Ursachen hierfür sind: unterschiedliche Örtlichkeiten, unterschiedliche Wettersituationen und Jahreszeiten, rauhe Meßbedingungen sowie fehlerhafte Datenübertragungen.

Die bei der Überwachung von Umweltprozessen anfallenden Datenmengen können nur durch fachgerechte Interpretation von Experten richtig ausgewertet werden. Nur der Fachmann kann letztendlich ein geeignetes Erkennungs- und Steuerungssystem dimensionieren und überwachen. Bedingt durch die Komplexität und die Vielfalt der zu berücksichtigenden Situationen kommen zur Bewältigung der oben angedeuteten Aufgaben immer mehr Multisensorsysteme zum Einsatz, deren Stärke in der Möglichkeit einer gegenseitigen Kompensation unterschiedlicher physikalischer bzw. chemischer Schwächen liegt. Ein weiterer Aspekt in diesem Zusammenhang besteht darin, daß durch den Einsatz von mehreren Sensoren, durch die jeweils eine relativ einfache Auswertung erfolgt, in vielen Fällen ein komplexer Sensor ersetzt werden kann, für den aufwendige Auswerteeinrichtungen erforderlich sind.

Aus der Druckschrift "Application of articial neural networks to an electronic olfactory system", measurement science & technology, May 1990, No. 5, p. 446-451, Bristol GB, Gardner et al., ist ein Analysesystem zur Klassifikation von Gaskomponenten in Gasgemischen bekannt, welches als elektronische Nase bezeichnet wird.

Hierbei werden mit Sensoren Meßwerte erfaßt, die in einem neuronalen Netzwerk verarbeitet werden, so daß am Ausgang des neuronalen Netzwerks Werte zwischen Null und Eins bereitstehen, aus denen auf das Vorhandensein einer Gaskomponente in dem Gasgemisch geschlossen werden kann.

Ein Verfahren zur Zustandsanalyse von Abwasser unter Verwendung von Sensoren und der Verarbeitung von Meßwerten in neuronalen Netzen ist ferner aus der DE 42 27 727 A1 bekannt.

Weitere Verfahren und Vorrichtungen zur Anwendung von neuronalen Netzen zur Verarbeitung von Meßwerten werden in der EP 0 432 267 A1, der EP 0 431 910 A1, der EP 0 521 643 A1, der Druckschrift "Detection of vapours and odours from multisensor array using pattern-recognition techniques", sensors and actuators, B9 (1992), Juli, Nr. 1, S. 9-15, Lausanne Ch, Gardner et al. (XP 000 29 7731), der EP 0 417 571 A1 und der Druckschrift "The basic ideas in neural networks", Communications of ACM, March 1994, Vol. 37, No. 3, S. 87-92, Rumelhart et al. beschrieben.

Ausgehend vom Stand der Technik und der vorstehend aufgezeigten Problematik liegt der Erfindung die Aufgabe zugrunde, ein verbessertes Analysegerät anzugeben, welches auf der Basis von auf komplizierte Weise miteinander verknüpften Daten, wie sie beispielsweise bei der Analyse von kommunalen Abwässern auftreten, mit vertretbarem Aufwand zuverlässige Analysenergebnisse liefert, die für eine automatische Steuerung komplexer Anlagen, wie z.B. Klärwerke geeignet ist.

Diese Aufgabe wird bei einem Analysegerät der eingangs angegebenen Art erfindungsgemäß dadurch gelöst, daß die Schaltungsanordnung jeweils eine jedem Sensor nachgeschaltete Schaltungseinheit zur Signalvorverarbeitung und Gewinnung zusätzlich extrahierter Merkmale jeweils ein dieser Schaltungseinheit nachgeschaltetes neuronales Netzwerk mit einem einzigen Ausgangsneuron, das eine lineare Übertragungscharakteristik aufweist, und einen den Ausgängen der neuronalen Netzwerke nachgeschalteten Fuzzy-Klassifikator umfaßt.

Es hat sich gezeigt, daß mit Hilfe eines neuronalen Netzwerks große Datenmengen, wie sie beispielsweise in einem Klärwerk anfallen, im Echtzeitbetrieb zur Gewinnung der für eine Anlagensteuerung erforderlichen Meßdaten, insbesondere in Verbindung mit einer Fuzzy-Logik, zuverlässig verarbeitet werden können.

Von besonderem Vorteil ist, daß mittels des Analysegeräts Online-Verfahren zur Messung von Nitrat, Ammonium, Phosphat, Chlor, Chlordioxid, Sauerstoffgehalt, pH-Wert, elektrischer Leitfähigkeit, Redoxwert, Trübung und ähnlichen Parametern ermöglicht wird, wobei auf den Einsatz von akademisch ausgebildetem Bedienungspersonal praktisch völlig verzichtet werden kann.

Darüber hinaus ist mittels des Analysegeräts eine robuste und sichere, weil verhältnismäßig einfache Messung im Gegensatz zu Messungen mit hochkomplexen empfindlichen Einzelanalysatoren möglich, bei denen die Klassifikation off-line erfolgt und das Ergebnis zum Teil erst einige Zeit (z.B. etwa eine Stunde) nach einer Probenentnahme verfügbar ist.

Durch die Verwendung mehrerer robuster Einzelsensoren ist das Analysegerät schließlich auch kostengünstig und technisch einfach herzustellen. Dies wiederum ermöglicht eine leichte Wartung sowie eine hohe Bedienerfreundlichkeit. Schließlich ist ein direktes und schnelles Einlernen des akademisch nicht ausgebildeten Bedienungspersonals möglich.

Weitere vorteilhafte Ausgestaltungen der Erfindung sind Gegenstand der Unteransprüche.

Die nachfolgende Beschreibung dient im Zusammenhang mit beiliegender Zeichnung der näheren Erläuterung.

Es zeigen:
- Fig. 1: schematisch eine Schaltungsanordnung zur Signalerkennung und -verarbeitung mittels mehrerer Sensoren;
- Fig. 2: detailliert schematisch eine Schaltung zur Signalvorverarbeitung;
- Fig. 3a: schematisch den prinzipiellen Aufbau eines Netzwerks für drei Eingangsmerkmale, umfassend zwei Neuronen in der verdeckten Schicht und ein Ausgangsneuron;
- Fig. 3b: beispielhaft eine sigmoide Funktion eines Neurons der verdeckten Schicht;
- Fig. 3c: schematisch eine lineare Übertragungscharakteristik eines Ausgangsneurons;
- Fig. 4: den prinzipiellen Aufbau eines Trübungssensors;
- Fig. 5: schematisch Intensitäts-Trübungskurven und
- Fig. 6: schematisch eine Schaltung zur Verarbeitung der Signalwerte.

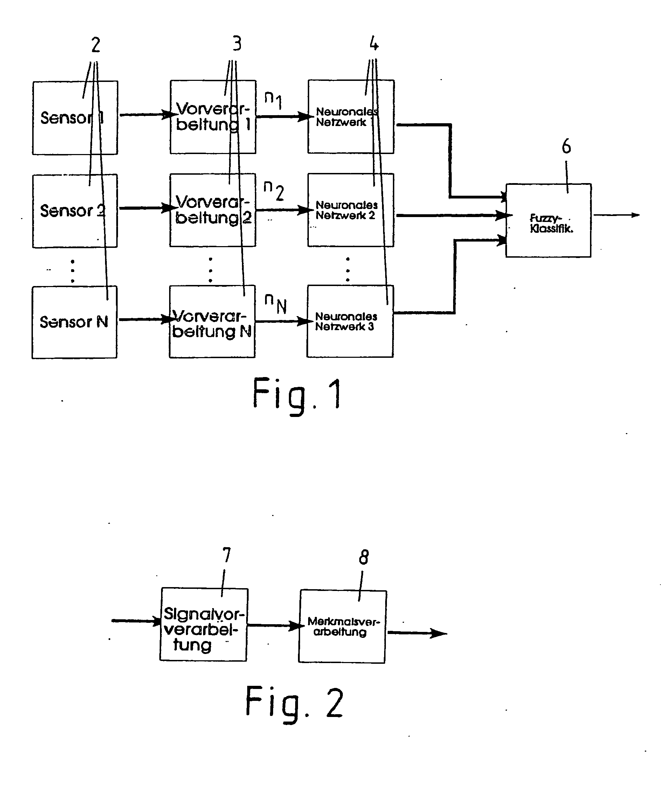

Wie aus Fig. 1 hervorgeht, welche eine Schaltungsanordnung zur Signalerkennung bestehend aus einer Kombination von N Sensoren 2 für den pH-Wert, die Trübung, den Redoxwert usw. darstellt, wird jedes Sensorsignal der N-Sensoren 2 mittels einer den Sensoren 2 jeweils nachgeschalteten Schaltungseinheit zur Signalvorverarbeitung 3 und einem dieser nachgeschalteten neuronalen Netz 4 verarbeitet und einem Fuzzy-Klassifikator 6 zugeführt, der schließlich über Fuzzyfizierung und De-Fuzzyfizierung den entsprechenden Nitratgehalt, Ammoniumgehalt, Phosphatgehalt oder einen ähnlichen Parameter (BOD, TOC u.dgl.) klassifiziert. Die Verarbeitung kann sowohl in analoger, digitaler als auch aus einer Kombination von analoger und digitaler Schaltungstechnik realisiert werden.

Beispielsweise kann der Klassifikator in Form einer 8-oder 16-Bit-Recheneinrichtung oder auch in Form einer Gleit-Recheneinrichtung ausgeführt sein.

Die Signalverarbeitung jedes Einzelsensors 2 umfaßt eine Signalverarbeitung 7 in Form einer spezifischen Signalglättung, wie z. B. lineare oder nichtlineare Filterung (IIR, FIR, Mediumfilter oder dgl.), einer Signalnormierung zur Anpassung auf einen Standardpegel und/oder einer Kalibrierung und eine Merkmalsverarbeitung (Merkmalsgewinnung) 8, wie es in Fig. 2 dargestellt ist.

Merkmale für die anschließende Klassifikation mittels eines neuronalen Netzwerks 4 können dabei entweder die geglätteten und/oder kalibrierten und/oder normierten Zeitsignale und/oder darüber hinaus aus den derart vorverarbeiteten Zeitsignalen zusätzlich extrahierte Merkmale in unterschiedlichen Kombinationen sein. Derartige extrahierte Merkmale können beispielsweise die Energieinhalte, die Leistung, verschiedene Mittelwerte, die spektrale Leistung, spektrale Leistungsdichten, Korrelationskoeffizienten, Kovarianzkoeffizienten, Steigungen, Maximal- und Minimalwerte, Krümmungen, Fourier-Deskriptoren u.dgl. sein. Alle Merkmale können über sensorspezifische, zeitlich äquivalente und/oder zeitlich unterschiedliche Fensterlängen ermittelt werden. Ebenso können alle erwähnten Merkmale auch direkt aus den in den Spektralbereich transformierten Zeitsignalen in ähnlicher oder gleicher Weise gewonnen werden. In diesem Falle beinhaltet die Signalvorverarbeitung 7 ein weiteres Modul einer Zeit-Frequenz-Transformation (nicht dargestellt). Dabei kann beispielsweise eine Fourier-Tansformation der reinen Sensorsignale, der geglätteten Signale, der normierten Signale, der kalibrierten Signale oder der Merkmale selbst sensorspezifisch ausgeführt werden. Die Transformation kann auf analoge Weise mittels einer digitalen Filterbank oder auf diskrete Weise mittels FFT oder DFT vorgenommen werden.

Die Merkmalsextraktion kann sowohl in analoger, digitaler als auch aus einer Kombination von analoger und digitaler Schaltungstechnik oder aus speziellen Hardwarebausteinen (Filterbausteinen oder Fourier-Transformatoren) realisiert werden. Alle Merkmalsextraktoren sind aus an sich bekannten Minimun- und Maximum-Bildnern, Differentiatoren, Integratoren, Summierern, Multiplizierern, Dividierern, logischen Verknüpfern, Transformationsnetzwerken, RAM-, ROM-Zellen oder dgl. oder aus einer Kombination derartiger Schaltungselemente aufgebaut (nicht dargestellt).

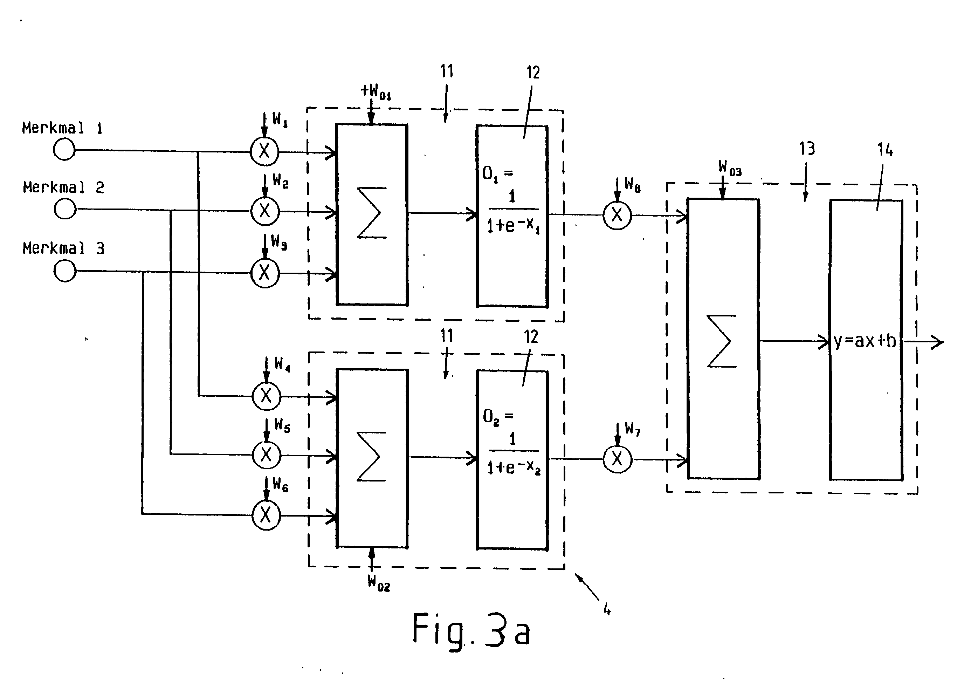

Das in Fig. 1 dargestellte zum Einzelsensor 2 gehörige neuronale Netzwerk 4 umfaßt im Falle der spezifischen Einzelsensorklassifikation soviele Netzwerkeingänge nᵢ, wie aus den jeweiligen zugehörigen Zeitsignalen oder Frequenzsignalen an Merkmalen ausgewählt wurden. Das zum jeweiligen Einzelsensor 2 gehörige neuronale Netzwerk 4 besteht somit sensorspezifisch aus nᵢ Eingängen, aus keiner, einer oder mehreren verdeckten Schichten, mit z.B. I Neuronen 11 (vgl. Fig. 3a) in der ersten verdeckten Schicht, J Neuronen 11 in der zweiten verdeckten Schicht und so fort, sowie einem Ausgangsneuron 13 (vgl. Fig. 3a), welches den jeweils spezifischen Sensorwert (z.B. Nitratgehalt in z.B. mg/l o.dgl.) ausgibt. Dabei kann die Anzahl der Neuronen 11 in den verdeckten Schichten jeweils unterschiedlich oder gleich sein.

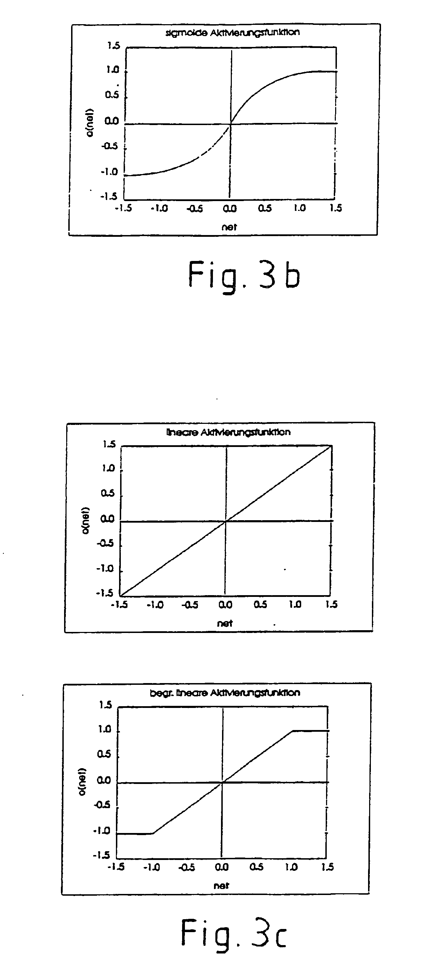

Grundsätzlich kann das neuronale Netzwerk 4 als im feed-forward-Modus arbeitendes Netzwerk ausgebildet sein, bei dem die Neuronen 11 der verdeckten Schicht eine Übertragungscharakteristik 12 wie in Fig. 3b dargestellt aufweisen. Das Ausgangsneuron 13 weist dagegen sensorspezifisch eine lineare Übertragungscharakteristik 14 mit Begrenzung oder ohne Begrenzung auf (Fig. 3c).

Wie aus Fig. 3a hervorgeht, welche beispielhaft den prinzipiellen Aufbau eines Netzwerks 4 für drei Eingangsmerkmale mit den beiden Neuronen 11 in der verdeckten Schicht und dem Ausgangsneuron 13 zeigt, besitzen dabei die Neuronen 11 in der verdeckten Schicht eine sigmoide Funktion 12, wie sie beispielhaft in Fig. 3b dargestellt ist, während das Ausgangsneuron 13 eine lineare Übertragungscharakteristik 14 je nach gewähltem Sensor mit einer Beschränkung des Wertebereichs von -a bis +a, beispielsweise von -1 bis +1, oder auch ohne Beschränkung des Wertebereichs besitzt (vgl. Fig. 3c). Biasgewichte W01, W02 und W03 können dabei jeweils gleich Null oder ungleich Null sein. Die Ausgabewerte dieser N neuronalen Netzwerke 4 aller eingesetzter Sensoren 2 werden sodann einem Fuzzy-Klassifikator zugeführt. Des weiteren können Schalteinrichtungen vorgesehen sein, mit deren Hilfe nach Abschluß eine Lernphase die sigmoide Übertragungscharakteristik 12 in einer verdeckten Schicht des neuronalen Netzwerks 4 gegen lineare Übertragungsfunktionen austauschbar ist.

Alle verwendeten neuronalen Netzwerke 4 oder Fuzzy-Klassifikatoren können jeweils durch elektronische Bauelemente realisiert sein. Diese Bauelemente können sowohl ein spezieller Fuzzy-Chip oder Neuro-Chip und/oder ein *µ*-Controller und/oder ein digitaler Signalprozessor (DSP) und/oder ein spezielles elektronisches Netzwerk in digitaler oder analoger Bauweise sein. Dabei können alle Funktionsblöcke auch in einem einzigen analogen oder digitalen Baustein zusammengefaßt sein.

Im folgenden wird beispielhaft die Verarbeitung des Signals eines Einzelsensors, in diesem Fall ein die Trübung bestimmendes Signal des Analysegeräts näher beschrieben.

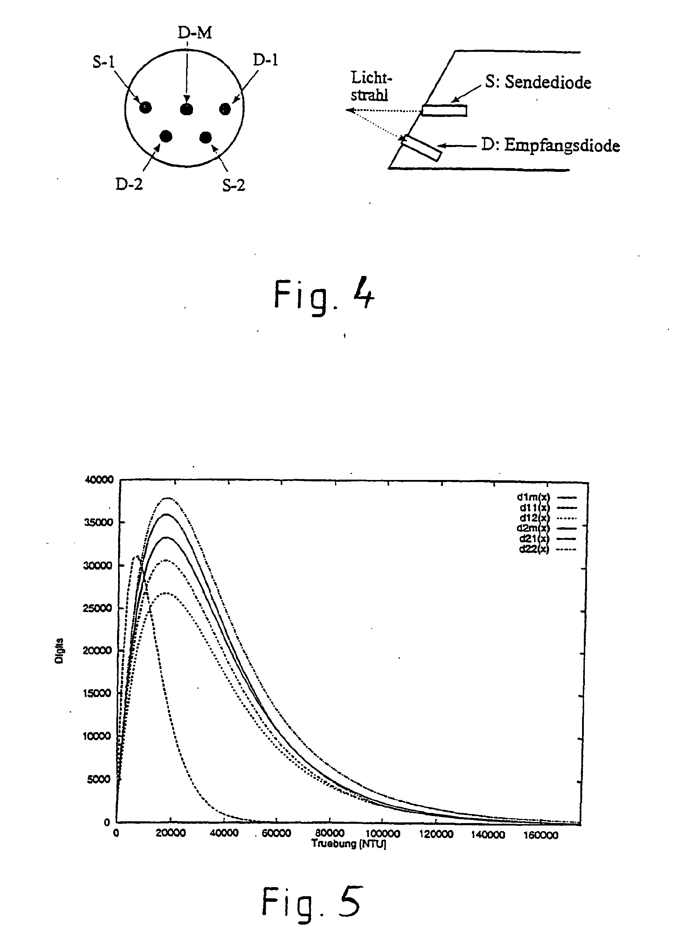

Wie aus Fig. 4 hervorgeht, welche den prinzipiellen Aufbau eines an sich bekannten Trübungssensors zeigt, ergeben sich entsprechend den unterschiedlich langen Ausbreitungswegen zwischen Sende- und Empfangsdioden (S1, S2, D1, D2, DM) Intensitäts-Trübungskurven, wie sie in Fig. 5 schematisch dargestellt sind.

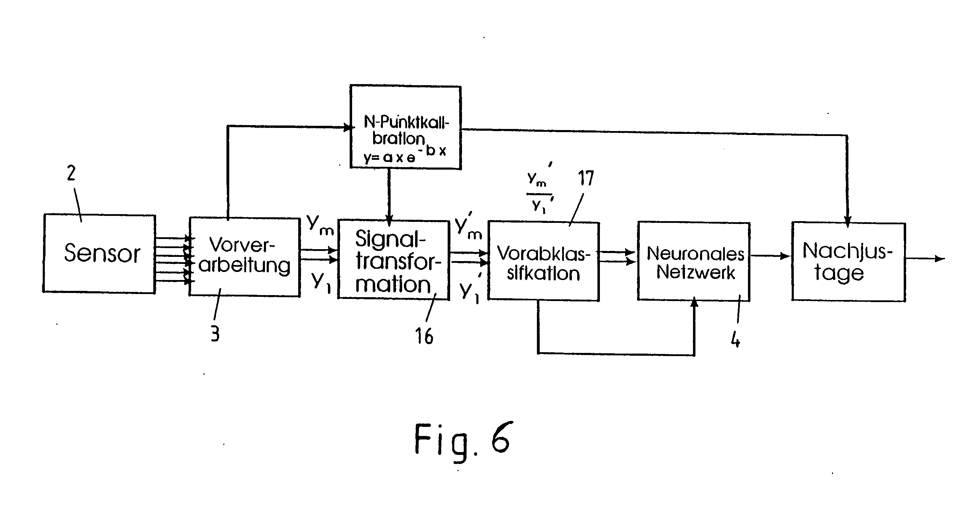

Bei den dargestellten beiden Sendedioden (S1, S2) und drei Empfangsdioden (D1, D2, DM) gibt es sechs unterschiedliche Ausbreitungswege und somit pro Trübungswert sechs unterschiedliche Werte. Die Verarbeitung der Signalwerte wird wie in Fig. 6 dargestellt durchgeführt. Die 6-Tupel von Intensitäts-Trübungs-Charakteristiken (die Intensitäten aller Ausbreitungswege) werden von einem möglicherweise vorhandenen Offset befreit und auf ein sensorspezifisches Referenzsignal normiert. Anschließend werden alle kurzen Signalwerte (135°-Streuungen) gemittelt. Die beiden so entstandenen Signalwerte (schlamm- und sensorabhängig, 135°-Signale und 90°-Signale) werden einer weiteren Transformation 16 zugeführt, die deren Werte auf dann schlamm- und sensorunabhängige Werte transformiert.

Mittels eines Vorab-Klassifikators 17 wird durch Verhältnisbildung der schlamm- und sensorunabhängigen Werte und Vergleich mit einem schlammspezifischen Vergleichswert (z.B. Komparator oder Operationsverstärkerschaltung) entschieden, ob man sich im Bereich hoher Trübung (beispielsweise > 4000 NTU) oder im Bereich niedriger Trübung (beispielsweise < 4000 NTU) befindet. Die Wahl der Entscheidungsschwelle zur Vorabklassifikation ist schlammspezifisch frei wählbar oder kann aus einer entsprechenden Transformation bezüglich einer N-Punktkalibration ermittelt werden. Anschließend werden die schlamm- und sensorunabhängigen Werte dem neuronalen Klassifikator (neuronales Netzwerk) 4 zugeführt, dem schlammunabhängige Trübungs-Intensitätskurven "eingelernt" worden sind.

Der auf diese Weise klassifizierte Trübungswert wird in einem weiteren Verarbeitungsschritt einem an sich bekannten abschnittsweisen linearen Interpolator zugeführt, der die mittels des neuronalen Netzwerks 4 klassifizierten Trübungswerte auf die bei der Kalibration verwendeten Kalibrationssollwerte hinsichtlich erhöhter Genauigkeit korrigiert. Als Interpolator können beispielsweise Tiefpaßfilter (analog oder digital), Mittelwertbildner oder ähnliches eingesetzt werden. Der Schritt der Korrektur des Trübungssollwertes kann bei weniger hohen Genauigkeitsanforderungen an das Meßergebnis bedarfsmäßig weggelassen werden. Je nach Vorabklassifikation werden in das Netzwerk 4 die für den hohen oder niedrigen Trübungsbereich charakteristischen Gewichte, Parameter, Übertragungscharakteristischen online eingespeist. Die schlamm- und sensorunabhängigen Werte werden durch Verhältnisbildung einer Vorabklassifikation zugeführt, die entscheidet, ob man sich in Substanzen hoher oder niedriger Trübung befindet.

Neben der bereits oben aufgeführten Verarbeitung ist es ebenso möglich, zur weiteren Erhöhung der Genauigkeit die fünf Werte für die kurzen Ausbreitungswege (135°-Streuungen) nicht zu mitteln und/oder Kombinationen aus den verschiedenen fünf Werten zu bilden, so daß sich letztlich ein N-dimensionaler Merkmalsvektor ergibt, wobei N ≥ 2 ist. Die weitere Verarbeitung geschieht entsprechend dem oben Ausgeführten. Je nach Entscheidung wird der N-dimensionale Merkmalsvektor einem neuronalen Netzwerk 4 mit den jeweiligen trübungsspezifischen Gewichten (hoher oder niedriger Trübungsbereich) zugeführt und klassifiziert.

Die Vorteile der Vorabklassifikation sind die Minimierung der Netzwerktopologie und die Erhöhung der Verarbeitungsgeschwindigkeit bei gleichzeitiger Erhöhung der Klassifikationsgüte der Trübungswerte.

Die Kalibrierung des Analysegeräts wird folgendermaßen durchgeführt. Der Trübungsintensitätsverlauf wird durch eine Exponentialfunktion (y1 = ax exp(-bx) bzw. y2 = ax exp/-bx(1-x/c) charakterisiert. Je nach Meßgenauigkeit und/oder Medium werden über ein N-Punktkalibrierverfahren Parameter a, b bzw. c bestimmt. Dabei muß N ≥ 2 sein. Entsprechend N wird der Sensor 2 in die N verschiedenen definierten Konzentrationen des Meßmediums eingebracht. Aus den so gemessenen Werten werden die Parameter nach der Methode des Minimalfehlerquadrates approximiert und/oder in geschlossener Form berechnet. Diese Approximation erfolgt entweder für alle und/oder mindestens zwei Trübungswerte. Anschließend werden die Trübungs-Intensitätskurven durch eine die Kalibrierparameter enthaltende Variablentransformation auf normierte Trübungs-Intensitätskurven transformiert, wie es oben beschrieben wurde. Beispielsweise lautet die variable Transformation für den gemittelten kurzen Ausbreitungsweg ym: ym' = bm/am ym und x' = bmx. Bei Verwendung von mehr als zwei Signalen werden über entsprechende mehrdimensionale Variablen-Transformationen die normierten Trübungs-Intensitätsverläufe ermittelt.

Das "Einlernverfahren" für das neuronale Netzwerk 4 erfolgt mittels einer an das Ausgangsneuron 13 mit linearer Übertragungscharakteristik angepaßter Backpropagationregel mit oder ohne eingebautem Begrenzer (Signumfunktionsglied) oder mittels radialer Basisfunktionen bei Netzwerken 4 mit keiner verdeckten Schicht. Aufgrund der vereinfachten Ableitung des Fehlers nach den Netzwerkgewichten kann das Lernverfahren ebenfalls hardwaremäßig analog oder digital auf an sich bekannte weise leicht realisiert werden.

Die oben beschriebenen Verfahren können sowohl mittels analoger als auch digitaler Schaltungen realisiert werden. Im Falle der Digitalverarbeitung kann die Signalverarbeitung nach entsprechender A-D-Wandlung in µ-Controllern, in speziellen Hardware-Bausteinen (Fuzzy-Chip, Neurochip) oder ähnlichen Hardwarebausteinen (z. B. ASIC) bzw. in Signalprozessoren mit unterschiedlicher Genauigkeit und Arithmetik verarbeitet werden, wobei die Analog-Digital-Schnittstelle mit entsprechender Wortgenauigkeit entsprechend den jeweiligen Anfordernissen an jeder beliebiger Stelle der verarbeitungskette erfolgen kann.

## Patentansprüche

1. Analysegerät zur Überwachung von Umweltprozessen umfassend mehrere Sensoren (2), deren Ausgangssignale jeweils einer Schaltungsanordnung zugeführt werden, **dadurch gekennzeichnet, dass** die Schaltungsanordnung jeweils eine jedem Sensor (2) nachgeschaltete Schaltungseinheit zur Signalvorverarbeitung und Gewinnung zusätzlich extrahierter Merkmale, jeweils ein dieser Schaltungseinheit nachgeschaltetes neuronales Netzwerk (4) mit einem einzigen Ausgangsneuron, das eine lineare Übertragungscharakteristik aufweist, und einen den Ausgängen der neuronalen Netzwerke (4) nachgeschalteten Fuzzy-Klassifikator (6) umfasst.

2. Analysegerät nach Anspruch 1, **dadurch gekennzeichnet, dass** zur Erzeugung der Sensormeßdaten mindestens zwei der folgenden Sensoren (2) vorgesehen sind: ein Sensor zur Erfassung der elektrischen Leitfähigkeit, ein pH-Wert-Sensor, ein Trübungssensor, ein Temperatur-sensor, ein Sauerstoffsensor und ein Sensor zur Erfassung des Redoxwertes, und dass das neuronale Netzwerk ausgebildet ist zur Ermittlung des Nitratgehalts und/oder des Phosphatgehalts und/oder des Ammoniumgehalts im Abwasser.

3. Analysegerät nach Anspruch 2, **gekennzeichnet durch** die Auswertung von mindestens zwei der folgenden Parametern: elektrische Leitfähigkeit, pH-Wert, Trübung, Sauerstoffgehalt, Redoxwert, Chlor und Chlordioxid, Nitrat, Ammonium und Phosphat mit Hilfe eines neuronalen Netzes (4) mit Neuronen (11) mit sigmoider Übertragungscharakteristik (12) in wenigstens einer verdeckten Schicht, und einem Ausgangsneuron (13) mit linearer Übertragungscharakteristik (14).

4. Analysegerät nach einem der Ansprüche 1 bis 3, **gekennzeichnet durch** eine hardwaremäßige Realisierung des Klassifikators in Form einer 8 Bit-Recheneinrichtung.

5. Analysegerät nach einem der Ansprüche 1 bis 3, **gekennzeichnet durch** eine hardwaremäßige Realisierung des Klassifikators in Form einer 16 Bit-Recheneinrichtung.

6. Analysegerät nach einem der Ansprüche 1 bis 5, **dadurch gekennzeichnet, dass** das neuronale Netzwerk (4) als im feed-forward-Modus arbeitendes Netzwerk ausgebildet ist, bei dem die Neuronen (11) der verdeckten Schicht eine Übertragungscharakteristik (12) mit eingeschränktem Wertbereich aufweisen.

7. Analysegerät nach Anspruch 6, **dadurch gekennzeichnet, dass** die Übertragungscharakteristik in der verdeckten Schicht eine sigmoide Übertragungscharakteristik (12) ist.

8. Analysegerät nach Anspruch 7, **dadurch gekennzeichnet, dass** die sigmoide Übertragungscharakteristik (12) nach Abschluss einer Lernphase gegen eine lineare Übertragungscharakteristik austauschbar ist.

9. Analysegerät nach einem der Ansprüche 1 bis 8, **dadurch gekennzeichnet, dass** Einrichtungen vorgesehen sind, mit deren Hilfe nach Abschluß einer Lernphase die sigmoide Übertragungs-charakteristik (12) in den verdeckten Schichten des neuronalen Netzwerks (4) gegen lineare Übertragungsfunktionen austauschbar sind.

## Claims

1. Analyser for monitoring environmental processes comprising a plurality of sensors (2), the output signals of which are in each case supplied to a circuit arrangement, **characterised in that** the circuit arrangement comprises in each case one circuit unit connected downstream of each sensor (2) and intended for signal preprocessing and obtaining additionally extracted features, in each case one neural network (4) connected downstream of this circuit unit and having a single output neurone with a linear transmission characteristic, and a fuzzy classifier (6) connected downstream of the outputs of the neural networks (4).

2. Analyser according to Claim 1, **characterised in that** at least two of the following sensors (2) are provided for generating the sensor measurement data: a sensor for detecting the electrical conductivity, a pH sensor, a turbidity sensor, a temperature sensor, an oxygen sensor and a sensor for detecting the redox value, and **in that** the neural network is designed to determine the nitrate content and/or the phosphate content and/or the ammonium content in the waste water.

3. Analyser according to Claim 2, **characterised by** the evaluation of at least two of the following parameters: electrical conductivity, pH value, turbidity, oxygen content, redox value, chlorine and chlorine dioxide, nitrate, ammonium and phosphate with the aid of a neural network (4) having neurones (11) with a sigmoid transmission characteristic (12) in at least one hidden layer, and an output neurone (13) with a linear transmission characteristic (14).

4. Analyser according to one of Claims 1 to 3, **characterised by** a hardware-based implementation of the classifier in the form of an 8-bit computing device.

5. Analyser according to one of Claims 1 to 3, **characterised by** a hardware-based implementation of the classifier in the form of a 16-bit computing device.

6. Analyser according to one of Claims 1 to 5, **characterised in that** the neural network (4) is designed as a network which operates in feed-forward mode and in which the neurones (11) of the hidden layer have a transmission characteristic (12) with a limited range of values.

7. Analyser according to Claim 6, **characterised in that** the transmission characteristic in the hidden layer is a sigmoid transmission characteristic (12).

8. Analyser according to Claim 7, **characterised in that** the sigmoid transmission characteristic (12) can be replaced by a linear transmission characteristic after completion of a learning phase.

9. Analyser according to one of Claims 1 to 8, **characterised in that** devices are provided, with the aid of which the sigmoid transmission characteristic (12) in the hidden layers of the neural network (4) can be replaced by linear transmission functions after completion of a learning phase.

## Revendications

1. Dispositif d'analyse pour la surveillance de processus environnementaux comprenant plusieurs capteurs (2) dont les signaux de sortie sont conduits à chaque fois à une disposition de circuit, **caractérisé en ce que** la disposition de circuit comporte à chaque fois une unité de circuit connecté en aval de chaque capteur pour le prétraitement des signaux et la production de caractéristiques extraits de façon complémentaire, à chaque fois un réseau neuronal (4) avec un seul neurone de sortie présentant une caractéristique de transmission linéaire, ledit réseau (4) étant connecté en aval de cette unité de circuit, et un classificateur fuzzy (6) connecté en aval des sorties des réseaux neuronaux.

2. Dispositif d'analyse selon la revendication 1, **caractérisé en ce que** l'on a prévu pour la production de données de mesure de capteur au moins deux des capteurs (2) suivants : un capteur pour détecter la conductivité électrique, un capteur de pH, un capteur d'opacité, un capteur de température, un capteur d'oxygène et un capteur pour détecter la valeur de réduction, et **en ce que** le réseau neuronal est réalisé pour déterminer la teneur en nitrate et / ou la teneur en phosphate et / ou la teneur en ammonium dans les eaux usées.

3. Dispositif d'analyse selon la revendication 2, **caractérisé par** l'évaluation d'au moins deux des paramètres suivants : conductivité électrique, le pH, l'opacité, la teneur en oxygène, valeur de réduction, chlore et dioxyde de chlore, nitrate, ammonium et phosphate au moyen d'un réseau neural (4) avec des neurones (11) avec une caractéristique de transmission sigmoïde (12) dans au moins une couche masquée et avec un neurone de sortie (13) avec une caractéristique de transmission linéaire (14).

4. Dispositif d'analyse selon une des revendications 1 à 3, **caractérisé par** une réalisation sous forme de hardware du classificateur en forme d'un dispositif de calcul à 8 bits.

5. Dispositif d'analyse selon une des revendications 1 à 3, **caractérisé par** une réalisation sous forme de hardware du classificateur en forme d'un dispositif de calcul à 16 bits.

6. Dispositif d'analyse selon une des revendications 1 à 5, **caractérisé en ce que** le réseau neuronal (5) est réalisé sous forme d'un réseau travaillant en mode prédictif , dans lequel réseau les neurones (11) de la couche masquée présentent une caractéristique de transmission (12) avec une zone de valeurs limitée.

7. Dispositif d'analyse selon la revendication 6, **caractérisé en ce que** la caractéristique de transmission dans la couche masquée est une caractéristique de transmission sigmoïde (12).

8. Dispositif d'analyse selon la revendication 7, **caractérisé en ce que** la caractéristique de transmission sigmoïde (12) peut être échangée après une phase d'apprentissage contre une caractéristique de transmission linéaire.

9. Dispositif d'analyse selon une des revendications 1 à 8, **caractérisé en ce que** l'on a prévu des installations grâce auxquelles on peut échanger la caractéristique de transmission sigmoïde (12) dans les couches masquées du réseau neural (4) après la fin d'une phase d'apprentissage contre des fonctions de transmission linéaires.
